# EUROPEAN PATENT SPECIFICATION

(11) **EP 2 585 608 B1**
(45) Date of publication and mention of the grant of the patent: **26.07.2017**
(21) Application number: 11728222.8
(22) Date of filing: 16.06.2011
(51) Int. Cl.: C12P 1/02, C12P 35/00, C12P 37/00, C12N 1/14

(54) **AIR BUBBLE FERMENTATION PROCESS**
LUFTBLASENGÄRUNGSVERFAHREN
PROCÉDÉ DE FERMENTATION À BULLES D'AIR

(30) Priority: 22.06.2010 EP 10166818
(43) Date of publication of application: 01.05.2013
(73) Proprietor: DSM Sinochem Pharmaceuticals Netherlands B.V., 2613 AX Delft (NL)
(72) Inventor: HOFMEESTER, Joseph, Johannes, Maria, NL-2289 BL Rijswijk (NL); WINDEN, VAN, Wouter, Adrianus, NL-2596 EJ Den Haag (NL)
(74) Representative: van Dam, Vincent
(86) International application number: PCT/EP2011/060053
(87) International publication number: WO 2011/161004

(56) References cited:
- WO-A1-01/25468
- US-A1- 2004 266 009
- KONIG B ET AL: "STRATEGIES FOR PENICILLIN FERMENTATION IN TOWER LOOP REACTORS", BIOTECHNOLOGY AND BIOENGINEERING, vol. 24, no. 2, 1982, pages 259-280, XP002613109, ISSN: 0006-3592
- Fu, Wenge: "Computer aided industrial reactor scale-up designs - Airlift bioreactors", , 3 November 2009 (2009-11-03), XP002661743, Retrieved from the Internet: URL:http://es.reocities.com/CapeCanaveral/ 4188/design.htm [retrieved on 2011-10-14]
- GBEWONYO K ET AL: "ENHANCING GAS LIQUID MASS TRANSFER RATES IN NON-NEWTONIAN FERMENTATIONS BY CONFINING MYCELIAL GROWTH TO MICRO BEADS IN A BUBBLE COLUMN", BIOTECHNOLOGY AND BIOENGINEERING, vol. 25, no. 12, 1983, pages 2873-2887, XP002613110, ISSN: 0006-3592 cited in the application
- AL-QODAH ZAKARIA: "Continuous production of antibiotics in an airlift fermentor utilizing a transverse magnetic field", APPLIED BIOCHEMISTRY AND BIOTECHNOLOGY, vol. 87, no. 1, April 2000 (2000-04), pages 37-55, XP002613111, ISSN: 0273-2289
- GAVRILESCU M ET AL: "Performance of airlift bioreactors in the cultivation of some antibiotic producing microorganisms", ACTA BIOTECHNOLOGICA, vol. 18, no. 3, 1998, pages 201-229, XP002613112, ISSN: 0138-4988
- ZHOU W ET AL: "Cephalosporin C production by a highly productive Cephalosporium acremonium strain in an airlift tower loop reactor with static mixers", JOURNAL OF BIOTECHNOLOGY, vol. 28, no. 2-3, 1 April 1993 (1993-04-01), pages 165-177, XP023705152, ELSEVIER SCIENCE PUBLISHERS, AMSTERDAM, NL ISSN: 0168-1656, DOI: 10.1016/0168-1656(93)90168-M [retrieved on 1993-04-01]
- PUNITA MISHRA ET AL: "A comparative evaluation of oxygen mass transfer and broth viscosity using Cephalosporin-C production as a case strategy", WORLD JOURNAL OF MICROBIOLOGY AND BIOTECHNOLOGY, vol. 21, no. 4, 1 June 2005 (2005-06-01), pages 525-530, XP019271578, KLUWER ACADEMIC PUBLISHERS, DO ISSN: 1573-0972
- SRIVASTAVA P AND KUNDU S: "Studies on cephalosporin-C production in an air lift reactor using different growth modes of Cephalosporium acremonium", PROCESS BIOCHEMISTRY, vol. 34, no. 4, 1 June 1999 (1999-06-01), pages 329-333, XP002613113, ISSN: 1359-5113, DOI: 10.1016/S0032-9592(98)00059-4
- KAWAGOE MIKIO ET AL: "Application of bubble column fermentors to submerged culture of Schizophyllum commune for production of L-malic acid", JOURNAL OF FERMENTATION AND BIOENGINEERING, vol. 84, no. 4, 1997, pages 333-336, XP002613114, ISSN: 0922-338X
- KAWAGOE M ET AL: "SUBMERGED CULTURE OF TRICHOLOMA MATSUTAKE MYCELIUM IN BUBBLE", JOURNAL OF BIOSCIENCE AND BIOENGINEERING, vol. 87, no. 1, 1 January 1999 (1999-01-01), pages 116-118, XP002974654, ELSEVIER, AMSTERDAM, NL ISSN: 1389-1723, DOI: 10.1016/S1389-1723(99)80020-6
- Burlingame R P and Verdoes J C: Biopharm International , vol. 19, no. 1 1 May 2006 (2006-05-01), pages 1-5, XP002613115, Retrieved from the Internet: URL:http://biopharminternational.findpharm a.com/biopharm/issue/issueDetail.jsp?id=94 60 [retrieved on 2010-12-07] cited in the application
- John E. Smith: "Chapter 10.3: Microorganisms as food" In: John E. Smith: "Biotechnology", 2009, Cambridge University Press, XP002661744, pages 178-184, page 181, paragraph 3 - page 182, paragraph 3; figure 10.6
- GARCIA-OCHOA F ET AL: "Bioreactor scale-up and oxygen transfer rate in microbial processes: An overview", BIOTECHNOLOGY ADVANCES, ELSEVIER PUBLISHING, BARKING, GB, vol. 27, no. 2, 1 March 2009 (2009-03-01), pages 153-176, XP025873361, ISSN: 0734-9750, DOI: 10.1016/J.BIOTECHADV.2008.10.006 [retrieved on 2008-11-12]
- LIU T ET AL: "Scale-up of L-lactic acid production by mutant strain Rhizopus sp. MK-96-1196 from 0.003 m<3> to 5 m<3> in airlift bioreactors", JOURNAL OF BIOSCIENCE AND BIOENGINEERING, ELSEVIER, AMSTERDAM, NL, vol. 101, no. 1, 1 January 2006 (2006-01-01), pages 9-12, XP025182963, ISSN: 1389-1723, DOI: 10.1263/JBB.101.9 [retrieved on 2006-01-01]

## Description

### Field of the invention

The present invention relates to the fermentative production of valuable compounds on an industrial scale whereby >50% of the total energy input is delivered by injection of an oxygen containing gas.

### Background of the invention

Many valuable compounds are manufactured by fermentative production in large, industrial scale fermentors in which a microorganism, which can be a filamentous fungus (such as *Penicillium chrysogenum* for compounds like β-lactams) produces the valuable compound under controlled conditions. In order to obtain a high productivity of the valuable compound in the broth, a high biomass concentration is required. Most fermentation processes are carried out as submerged fermentations with stirring and aeration. Aeration is necessary in order to provide the necessary oxygen for the microorganism and stirring is necessary in order to ensure good mixing of the broth and the air bubbles so as to achieve efficient oxygen transfer from the injected air into the broth. The efficiency of the oxygen transfer is decreased by high viscosity of the broth, which is caused by the high biomass concentrations in the broth and/or by the morphology of the microorganism used. Inefficient mixing results in reduced oxygen transfer with reduced biomass formation and reduced valuable compound production as a result. In most common processes, in particular for the production of β-lactams such as penicillin G, approximately 30-40% of the energy input is derived from air injection and some 60-70% from mechanical stirring. Both stirring and injection of air introduces a lot of energy into the fermentor which adds to the heat generated by the growing microorganisms and usually necessitates (forced) cooling in order to maintain the temperature of the fermentation process at the desired value.

In the last 50 years, much research efforts have focused on improving the fermentation processes e.g. for β-lactams, with a special focus on penicillin G. The period 1950-1984 has been reviewed by G.J.M Hersbach, C.P van der Beek and P.W.M van Dijk (The penicillins: properties, biosynthesis and fermentation. In: E.J Vandamme, Editor, Biotechnology of industrial antibiotics, Marcel Dekker, New York (1984), 45-140). The maximum oxygen transfer was increased by increasing the agitation power and the aeration rate. The disadvantage of these measures is that more energy is introduced which also requires improved cooling capacity and which therefore increases the cost price of the product (Hersbach *et al.,* page 95). Alternatively, strains and/or fermentation conditions could be selected which gave a lower viscosity broth (Hersbach *et al.,* page 51, paragraph 4).

More recently, Burlingame and Verdoes (BioPharm Int., 2006, 19(1), 1-5) isolated low-viscosity mutants of *Chrysosporium lucknowense* which showed a morphology characterized by mycelial fragmentation and the formation of discrete elements designated as "propagules". Cultures of these propagules exhibited lower viscosity, better nutrient and oxygen transfer and higher protein production. When compared to the parent strains, the low-viscosity mutant produced about twice the total protein yield when the two strains were grown under similar conditions. The low-viscosity strain afforded even improved culture conditions which, when applied, resulted in an additional 3-fold increase in protein production.

Viscosity reduction has also been obtained by altering feeding strategies in fermentors. For instance, Bhargava et al. (Biotechnol. Bioeng., 2003, 82, 111-117), demonstrated that a pulse-feed strategy of a limiting carbon source reduced the viscosity and mean mycelial particle size in fermentations with a strain of *Aspergillus bryzae* producing recombinant glucoamylase. These changes were accompanied by an increase in glucoamylase production by those strains.

Another solution for the high viscosity has been found in the use of immobilized cells in fermentation processes. There are numerous disclosures in the prior art whereby the fermentation has been carried out with immobilized cells in shake flasks, small scale laboratory fermentors, bubble columns and air lift fermentors, see B. Konig et al. (Biotechnol. Bioeng., 1982, 24(2), 259-280), Gbewonyo et al. (Biotechnol. Bioeng., 1983, 25(12), 2873-2887), Al-Qodah Zakaria (Appl. Biochem. Biotechnol., 2000, 87(1), 37-55), M. Gavrilescu et al. (Acta Biotechnologica, 1998, 18(3), 201-229), W. Zhou et al. (J. Biotechnol., 1993, 28(2-3), 165-177), Punita Mishra et al. (World J. Microbiol. Biotechnol., 2005, 21(4), 525-530), P. Srivastava et al. (Process Biochem., 1999, 34(4), 329), M. Kawagoe et al. (J. Ferm. Bioeng., 1997, 84(4), 333-336) and M. Kawagoe et al. (J. of Bioscience and Bioeng., 1999, 87(1), 116-118). For example, Gbewonyo et al (Biotechnol. Bioeng., 1983, 25(12), 2873-2887) demonstrated that by immobilizing *Penicillium chrysogenum* and confining the mycelial growth to micro beads, penicillin production in a 3 liter bubble column was much improved compared to the freely suspended mycelial structures. It has been suggested by the authors that when the cells are grown in the form of spherical pellets, the culture broth remains less viscous and capable of maintaining better gas-liquid mass transfer properties.

However, a disadvantage of the use of immobilized cells is that growth of the cells is decreased. As a consequence also the levels of product in the fermentation broth are very low. In the specific example of Gbewonyo *et al.,* only 5.5 g of penicillin G per liter fermentation broth was obtained. Although this value was 10 times higher than the comparative experiment with suspended cells in the bubble column, it is much lower than the levels obtained during industrial production of penicillin G in stirred and aerated fermentors (>25 g/1).

Moreover, it is well known to the skilled person that productivities obtained in fermentors at small scale are frequently not fully representative for those obtained at a larger scale, especially when there is a certain degree of viscosity. For example, mixing times on large scale are often much longer than on small scale, which can lead to the existence of gradients in the concentrations of nutrients that limit productivity. Suggestions presented in the prior art demonstrated on a small scale are therefore not necessarily successful when applied to large scale fermentations, such as fermentations at 10 m³ scale or larger. The prior art has never disclosed nor suggested that industrial scale viscous fermentation processes for valuable compounds, which normally are carried out in stirred, aerated fermentors, could advantageously be carried out in bubble columns at high biomass concentration.

### Detailed description of the invention

In a first aspect, the invention provides a process for the production of a compound in a bubble columns using an algae, or a fungus capable of producing said compound wherein the volume of the reaction medium is from 10 m³ to 5000 m³ and the viscosity of the reaction medium is from 10 centipoise to 200 centipoise, characterized in that >50% of the total energy input is delivered by injection of an oxygen containing gas and ≤50% of the total energy input is delivered by mechanical means and said injection of an oxygen containing gas is at a pressure corrected superficial gas velocity of between 4 cm/sec and 30 cm/sec. Preferably, ≥75% and more preferably ≥95% of the total energy input is delivered by injection of an oxygen containing gas and ≤50%, preferably ≤25%, more preferably ≤5% of the total energy input is delivered by mechanical means. In a most preferred embodiment, 100% of the total energy input is delivered by injection of an oxygen containing gas and no energy input is delivered by mechanical means. The process of the invention comprises a bubble column which is characterized in that mixing of the content of the fermentor is effectuated by the injection of the oxygen containing gas. The advantage of the present invention is that by reducing or replacing mechanical means for energy input by injection of an oxygen containing gas, the yield of valuable compound on energy, for example defined as amount of β-lactam produced per amount of energy consumed in the fermentation process, is increased.

In the context of the present invention, the term "total energy input" is defined as the combined energy introduced into the fermentor by either the compressed gas or mechanical means and is excluding chemical energy or heat introduced by the gas or liquids that are fed to the fermentor.

In the context of the present invention, the term "mechanical means" is defined herein as any means capable of direct input of energy into the liquid phase, *i.e.* not indirectly via a gas phase. A suitable means is a stirring device which is well known the person skilled in the art. Stirring devices for fermentors are amply documented in the art and commercially widely available.

In the context of the present invention, the term "bubble column" refers to an apparatus used for gas-liquid reactions consisting of vertically arranged cylindrical columns that can be built in numerous forms of construction. The introduction of gas takes place at the lower half of the column and causes a turbulent stream to enable an optimum gas exchange. Mixing occurs through gas sparging and displacement and entrainment of the liquid by rising gas bubbles. Bubble columns are characterized by a high liquid content and a moderate phase boundary surface and are used in various types of fermentations.

"Industrial scale" is defined herein as a fermentation process carried out in a fermentor which is having a volume of ≥10 m³, preferably ≥25 m³, more preferably ≥50 m³, most preferably ≥100 m³, preferably less than 5000 m³. Alternatively, industrial scale may be defined herein as a fermentor having a height of at least 4 meters, more preferably of at least 5 meters, more preferably of at least 6 meters, more preferably of at least 7 meters, more preferably of at least 8 meters, more preferably of at least 9 meters, more preferably of at least 10 meters. Preferably, the fermentor has a height of less than 15 meters. Industrial scale may also be defined as that the concentration of the product, such as a fatty acid or a β-lactam, at the end of the fermentation in the broth is at least 5 g/l, preferably at least 10 g/l, more preferably at least 15 g/l, more preferably at least 20 g/l and most preferably at least 25 g/l.

The oxygen containing gas may be any oxygen containing gas, such as air, oxygen-enriched air, pure oxygen or mixtures thereof. Preferably the oxygen containing gas is air. Optimal results are obtained when the oxygen containing gas is released in the lower half of the fermentor at a pressure corrected superficial gas velocity between 4 cm/sec and 30 cm/sec, preferably between 7 cm/sec and 25 cm/sec, more preferably between 10 cm/sec and 20 cm/sec.

The process of the invention is suitable for the fermentative production of any valuable compound of interest, including primary or secondary metabolites, pharmaceutical proteins or peptides, or industrial enzymes. Primary metabolites are biomolecules that are essential to the growth, development or reproduction and are shared by many species. Primary metabolites are for example intermediates of the main metabolic pathways such as the glycolytic pathway or the TCA cycle. Examples of primary metabolites are amino acids and nucleic acids. Secondary metabolites are not essential for growth, development, or reproduction, but instead have an ecological function. Examples of secondary metabolites are antibiotics or β-lactams, especially β-lactam antibiotics. A preferred valuable compound is a β-lactam. Examples of β-lactams are clavulanic acid, penicillin (e.g. penicillin G, penicillin V or 6-aminopenicillinic acid) and semi synthetic penicillins such as amoxicillin and cephalosporins such as cephalosporin C. In a highly preferred embodiment the invention provides a process for the production of penicillin G using a *Penicillium chrysogenum* strain capable of producing penicillin G and furthermore characterized in that 100% of the total energy input is delivered by injection of an oxygen containing gas and that the fermentor is a bubble column.

Other preferred embodiments are processes for the production of a β-lactam wherein the β-lactam is an N-acylated derivative of β-lactam intermediates such as 7-amino-3-carbamoyloxymethyl-3-cephem-4-carboxylic acid (7ACCCA), 7-aminocephalosporanic acid (7-ACA), 7-amino-3-chloro-3-cephem-4-carboxylic acid (7-ACCA), 7-aminodesacetoxy-cephalosporanic acid (7-ADCA), 7-aminodesacetylcephalosporanic acid (7-ADAC), 7-amino-3-[(*Z*/*E*)-1-propen-1-yl]-3-cephem-4-carboxylic acid (7-PACA) and the like. The acyl group at the 7-amino position is preferably adipic acid yielding the corresponding adipoyl derivate as disclosed in WO 93/05158, WO 93/08287 or WO 2004/106347. Alternative suitable side chains have been disclosed in WO 95/04148, WO 95/04149, WO 96/38580, WO 98/48034 and WO 98/48035.

A suitable microbial strain for the process of the invention may be any wild type algal or fungal strain producing a valuable compound of interest. Furthermore, a suitable microbial strain of the invention may be a algal or fungal strain which has been obtained and/or improved by subjecting a parent or wild type strain of interest to a classical mutagenic treatment or to recombinant DNA transformation. The microbial strain may include filamentous and non-filamentous strains.

A preferred bacterium is an Actinomycete. Preferably, the Actinomycete is *Streptomyces clavuligerus*, which preferably produces clavulanic acid as the valuable compound. A fungus is preferably selected from the group consisting of Aspergillus, Trichoderma, Penicillium and Acremonium. Preferred examples are *Penicillium chrysogenum* for the production of penicillin G or penicillin V, *Acremonium chrysogenum* for the production of cephalosporin C and Aspergilli such as *Aspergillus niger* or *Aspergillus oryzae* either as wild type or classically improved strains producing, or genetically modified to over express genes encoding, enzymes such as amylases, lipases, phospholipases, galactolipases, hemicellulases, xylanases, cellulases, proteases and other enzymes known to be used in industry.

In a preferred embodiment the fungus is *Penicillium chrysogenum* and the secondary metabolites are adipoyl-7-ADCA, adipoyl-7-ACA, adipoyl-7-ADAC, adipoyl-7-ACCA, adipoyl-7-PACA or adipoyl-7-ACCCA, most preferred is adipoyl-7-ADCA. As disclosed in WO 93/05158, the *Penicillium chrysogenum* strain is transformed with and expressing a gene encoding an expandase. This engineered *Penicillium chrysogenum* strain, when grown in the presence of adipic acid as the side chain precursor in the fermentation vessel, produces and excretes adipoyl-7-ADCA.

Examples of penicillins that can be produced by the fermentation process of the invention are penicillin G, penicillin V or amoxicillin as disclosed in WO 2008/040731 and many others. Examples of cephalosporins are cephalosporin C but preferably N-acyl-derivatives of 7-ADCA as mentioned above.

It was unexpectedly found in a fermentation process at industrial scale where 100% of the total energy input was delivered by injection of an oxygen containing gas and no energy input was delivered by mechanical means that high productivities are obtained even at relatively high viscosity. Hence, a suitable viscosity in the reaction medium is from 5 centipoise to 500 centipoise, preferably from 10 centipoise to 200 centipoise, more preferably from 15 centipoise to 150 centipoise, most preferably from 25 centipoise to 75 centipoise. In order to have sufficient mixing of the broth in the fermentor as well as oxygen transfer from the injected oxygen containing gas (such as air) to the broth, the viscosity of the broth should not be too high and is preferably below 80 centipoise. Viscosity is generally determined mainly by two factors, *i.e.* the concentration of the biomass in the fermentation broth and by the morphology of the microorganism (called "biomass-specific viscosity").

The process of the present invention is not related to and therefore excluding the production of baker's yeast. Industrial production of baker's yeast is performed in aerated bubble columns without stirring. However, in this process the biomass produced per se is the purpose of the production process and no valuable compound is produced by the baker's yeast according to the process of the present invention.

The process of the present invention may be illustrated with, but is not limited to, the following proposed example.

A 100 m³ stirred tank fermentor with normal open pipe blower and Rushton turbine is retrofitted to determine the effect of reduced power input by the stirrer in a viscous fungal fermentation process. As it is expected that lowering the power input will decrease the bubble dispersion at the bottom of the fermentor, the blower is replaced by a blower that distributes the air over the entire surface area of the bottom of the fermentor. The power input by the stirrer is changed by changing the stirrer speed. The air flow rate is kept constant. In the situation where no power input was done by stirring, the stirrer is removed from the vessel.

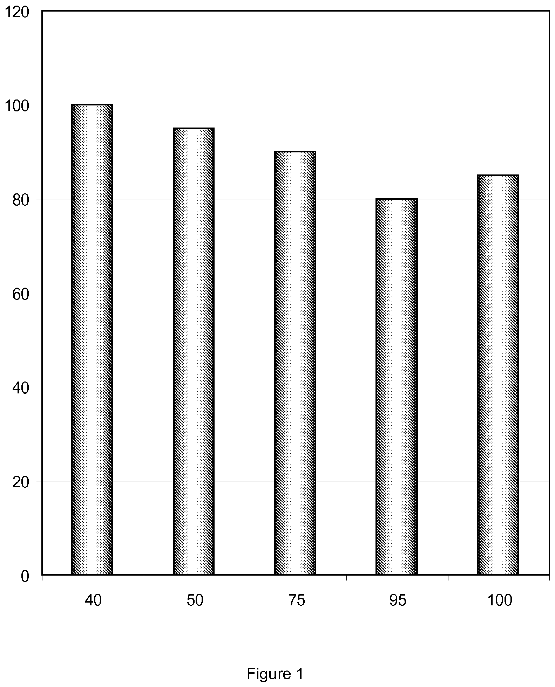

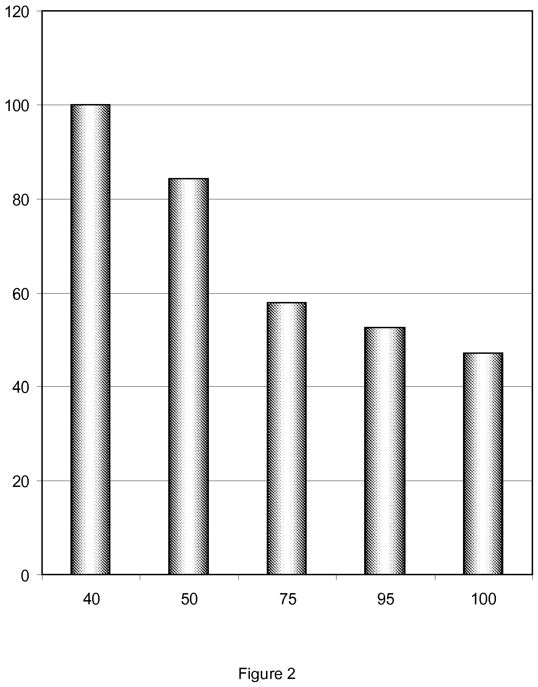

Penicillin fermentations with *Penicillium chrysogenum* with a viscosity increasing from 10 to 200 centipoise are performed with various ratios (40, 50, 75, 95 and 100%) of power input by the airflow relative to stirring to determine the effect of the stirrer power input. This ratio is achieved during the stage of the fermentation when oxygen transfer is limiting the production. The results are given in Figures 1 and 2.

The amount of product reduces when the power input by the impeller is reduced, because the oxygen transfer is reduced. It increases in the last step from 95 to 100% power input by air. This effect is expected to be caused by the removal of the stirrer, of which the presence and slow movement may hinder the bubble flow in the fermentor. The power input is reduced much more than the production, so the required amount of power per product is reduced (see Figure 2).

These figures show that the optimal effect is found when all the power is put in by aeration. This leads to a reduction of total costs per amount of product, and a smaller carbon footprint per amount of product.

### Legend to the Figures

Figure 1 displays the amount of product produced per fermentation in a penicillin fermentation with *Penicillium chrysogenum.* The X-axis is the amount of power input by air (% of total power input). The Y-axis is the amount of product produced (relative to a stirred tank fermentation).
Figure 2 displays the specific power consumption in a penicillin fermentation with *Penicillium chrysogenum* while changing stirrer speed. The X-axis is the amount of power input by air (% of total power input). The Y-axis is the amount of energy consumed (relative to a stirred tank fermentation).

## Claims

1. Process for the production of a compound in a bubble column using an algae or a fungus capable of producing said compound wherein the volume of the reaction medium is from 10 m³ to 5000 m³ and the viscosity of the reaction medium is from 10 centipoise to 200 centipoise, **characterized in that** ≥50% of the total energy input is delivered by injection of an oxygen containing gas and ≤50% of the total energy input is delivered by mechanical means and said injection of an oxygen containing gas is at a pressure corrected superficial gas velocity of between 4 cm/sec and 30 cm/sec.

2. Process according to claim 1 wherein ≥75% of the total energy input is delivered by injection of an oxygen containing gas and ≤25% of the total energy input is delivered by mechanical means.

3. Process according to claim 1 wherein 100% of the total energy input is delivered by injection of an oxygen containing gas.

4. Process according to any one of claims 1 to 3 wherein said injection of an oxygen containing gas is at a pressure corrected superficial gas velocity of between 7 cm/sec and 25 cm/sec.

5. Process according to any one of claims 1 to 4 wherein said compound is a β-lactam.

6. Process according to any one of claims 1 to 5 wherein said algae or fungus is an Acremonium species or a Penicillium species.

7. Process according to any one of claims 5 to 6 wherein said compound is adipoyl-7-ACA, adipoyl-7-ADCA or penicillin G.

## Patentansprüche

1. Verfahren zur Herstellung einer Verbindung in einer Blasensäule unter Verwendung einer Alge oder eines Pilzes die/der in der Lage ist, diese Verbindung herzustellen, wobei das Volumen des Reaktionsmediums 10 m³ bis 5.000 m³ beträgt und die Viskosität des Reaktionsmediums 10 Centipoise bis 200 Centipoise beträgt,
**dadurch gekennzeichnet, dass** ≥50% des gesamten Energieeintrags durch Einführen eines sauerstoffhaltigen Gases bereitgestellt wird und ≤50% des gesamten Energieeintrags durch mechanische Mittel bereitgestellt wird und das Einführen eines sauerstoffhaltigen Gases bei einer druckkorrigierten Oberflächen-Gasgeschwindigkeit von zwischen 4 cm/s und 30 cm/s erfolgt.

2. Verfahren gemäß Anspruch 1, wobei ≥75% des gesamten Energieeintrags durch Einführen eines sauerstoffhaltigen Gases bereitgestellt wird und ≤25% des gesamten Energieeintrags durch mechanische Mittel bereitgestellt wird.

3. Verfahren gemäß Anspruch 1, wobei 100% des gesamten Energieeintrags durch Einführen eines sauerstoffhaltigen Gases bereitgestellt wird.

4. Verfahren gemäß einem der Ansprüche 1 bis 3, wobei das Einführen eines sauerstoffhaltigen Gases bei einer druckkorrigierten Oberflächen-Gasgeschwindigkeit von zwischen 7 cm/s und 25 cm/s erfolgt.

5. Verfahren gemäß einem der Ansprüche 1 bis 4, wobei diese Verbindung ein β-Lactam ist.

6. Verfahren gemäß einem der Ansprüche 1 bis 5, wobei die Alge oder der Pilz eine Acremonium-Spezies oder eine Penicillium-Spezies ist.

7. Verfahren gemäß einem der Ansprüche 5 bis 6, wobei diese Verbindung Adipoyl-7-ACA, Adipoyl-7-ADCA oder Penicillin G ist.

## Revendications

1. Procédé pour la production d'un composé dans une colonne à bulles utilisant une algue ou un fongus capable de produire ledit composé, dans lequel le volume du milieu de réaction est de 10 m³ à 5000 m³ et la viscosité du milieu de réaction est de 10 centipoises à 200 centipoises, **caractérisé en ce que** ≥50 % de l'intrant énergétique total est délivré par injection d'un gaz contenant de l'oxygène et ≤50 % de l'intrant énergétique total est délivré par des moyens mécaniques et ladite injection d'un gaz contenant de l'oxygène est à une vitesse superficielle du gaz à pression corrigée entre 4 cm/s et 30 cm/s.

2. Procédé selon la revendication 1, dans lequel ≥75 % de l'entrant énergétique total est délivré par injection d'un gaz contenant de l'oxygène et ≤25 % de l'entrant énergétique total est délivré par des moyens mécaniques.

3. Procédé selon la revendication 1, dans lequel 100 % de l'entrant énergétique total est délivré par injection d'un gaz contenant de l'oxygène.

4. Procédé selon l'une quelconque des revendications 1 à 3, dans lequel ladite injection d'un gaz contenant de l'oxygène est à une vitesse superficielle du gaz à pression corrigée entre 7 cm/s et 25 cm/s.

5. Procédé selon l'une quelconque des revendications 1 à 4, dans lequel ledit composé est un β-lactame.

6. Procédé selon l'une quelconque des revendications 1 à 5, dans lequel ladite algue ou ledit fongus est une espèce d'Acremonium ou une espèce de Penicillium.

7. Procédé selon l'une quelconque des revendications 5 à 6, dans lequel ledit composé est l'adipoyl-7-ACA, l'adipoyl-7-ADCA ou la pénicilline G.
